# EUROPÄISCHE PATENTSCHRIFT

(11) **EP 4 277 598 B1**
(45) Veröffentlichungstag und Bekanntmachung des Hinweises auf die Patenterteilung: **26.03.2025**
(21) Anmeldenummer: 22702169.8
(22) Anmeldetag: 14.01.2022
(51) Int. Cl.: A61K 9/00

(54) **ORALER DÜNNFILM MIT PVA-TRIS-PUFFERSCHICHT**
ORAL THIN FILM COMPRISING A PVA-TRIS BUFFER LAYER
FILM MINCE BUCCAL COMPRENANT UNE COUCHE DE TAMPON PVA-TRIS

(30) Priorität: 15.01.2021 DE 102021100780
(43) Veröffentlichungstag der Anmeldung: 22.11.2023
(73) Patentinhaber: LTS Lohmann Therapie-Systeme AG, 56626 Andernach (DE)
(72) Erfinder: LINN, Michael, 55596 Waldböckelheim (DE); FICKER, Mario, 53179 Bonn (DE); NORELLI, Claudia, 56204 Hillscheid (DE); MÜLLER, Markus, 53840 Troisdorf (DE); SCHMITZ, Christoph, 56598 Rheinbrohl (DE)
(74) Vertreter: Held, Stephan
(86) Internationale Anmeldenummer: PCT/EP2022/050794
(87) Internationale Veröffentlichungsnummer: WO 2022/152878

(56) Entgegenhaltungen:
- WO-A1-2009/124096
- WO-A1-2009/124896

## Beschreibung

Die vorliegende Erfindung betrifft einen oralen Dünnfilm (auch als oral thin film oder unter der Abkürzung OTF bekannt), umfassend mindestens eine Schicht, die mindestens einen Polyvinylalkohol und Tris(hydroxymethyl)aminomethan in einer Menge von 15 bis 70 Gew.-%, bezogen auf das Gesamtgewicht der mindestens einen Schicht, enthält, und diesen oralen Dünnfilm zur Verwendung als Arzneimittel.

WO 2009/124096 A1 offenbart ein Pflaster, ein System und ein Verfahren zur Abgabe einer Permeationszusammensetzung an einen Patienten.

Polyvinylalkohol (PVA) ist als Matrixpolymer in oralen Dünnfilmen bekannt und weit verbreitet, da sich PVA gut als Matrixpolymer für Schaumformulierungen oder andere filmförmige OTF-Formulierungen eignet. PVA lässt sich jedoch nur gut in Lösungen mit geringer lonenstärke verarbeiten. Bei hohen Salzkonzentrationen, z.B. hohen Pufferkonzentrationen, fällt das PVA leicht aus oder verklumpt. Ein einheitliches Beschichtungsbild ist dann nicht mehr möglich. Für viele Anwendungen, wie beispielsweise die pH-Einstellung des Mundspeichels auf einen optimalen pH-Wert für Wirkstoffpermeation und Mundhygiene etc., ist der Einsatz stärkerer Puffer, also eine höhere Pufferkonzentration, notwendig um eine bestimmten pH-Wert im Speichel einzustellen bzw. ihn auf einem bestimmten pH-Wert zu halten, wie dies z.B. für eine konstante Wirkstoffaufnahme vorteilhaft ist.

Die Aufgabe der vorliegenden Erfindung besteht darin, vorstehend genannte Nachteile des Standes der Technik zu beheben. Insbesondere besteht die Aufgabe der vorliegenden Erfindung darin, einen oralen Dünnfilm bereitzustellen, der PVA umfasst und der eine möglichst hohe Menge an mindestens einer Puffersubstanz enthält, wobei die Formulierung gleichzeitig gut bearbeitbar sein soll, und das PVA nicht ausfällt und/oder verklumpt. Gleichzeitig, soll eine Formulierung gefunden werden, die gut geschäumt werden kann und die einen Film von möglichst hoher optische Homogenität bilden kann. Ferner soll durch den oralen Dünnfilm die Einstellung des pH-Wertes im Speichel des Patienten in einem möglichst großen Bereich möglich und dabei möglichst stabil sein. Dazu sollte möglichst viel Puffersubstanz in das PVA eingearbeitet werden können.

Zudem soll der orale Dünnfilm möglichst einfach herstellbar sein und auch einen möglichst einfachen Aufbau von mehrschichtigen oralen Dünnfilmen erlauben.

Obige Aufgabe wird durch einen mehrschichtigen oralen Dünnfilm nach Anspruch 1 gelöst, insbesondere durch einen oraler Dünnfilm, umfassend mindestens eine Schicht, die mindestens einen Polyvinylalkohol und Tris(hydroxymethyl)amino-methan (TRIS) in einer Menge von 15 bis 70 Gew.-%, bezogen auf das Gesamtgewicht der mindestens einen Schicht, enthält.

Ein oraler Dünnfilm, umfassend mindestens eine Schicht, die mindestens einen Polyvinylalkohol und Tris(hydroxymethyl)aminomethan (TRIS) enthält, wird im Folgenden auch als TRIS-PVA Schicht oder als TRIS-Pufferschicht bezeichnet.

Ein derartiger oraler Dünnfilm hat den Vorteil, dass sich TRIS prozentual und molar mit dem höchsten Anteil Puffer/PVA einarbeiten lässt. Bei anderen Salzen/Puffern (Phosphaten, Carbonaten, Zitronensäure etc.) kommt es hingegen zum Verklumpen bzw. Ausfallen von PVA.

Die TRIS-PVA Zusammensetzungen können gut geschäumt werden und bilden einen Film von hoher optischer Homogenität.

Es können auch Schichten mit sehr hoher TRIS Beladung hergestellt werden, vorzugsweise mit bis zu 50 Gew.-% TRIS. Zudem kann ein größerer pH-Bereich durch Einstellung mit Säure (z.B. HCI) abgedeckt werden. Ein weiterer Vorteil ist, dass sich diese oralen Dünnfilme in manchen Ausführungsformen ohne weiteren Kleber zusammenlaminieren lassen, d.h. es ist möglich relative dicke mehrschichtige Filme herzustellen, indem mehrere Lagen TRIS-PVA Schichten zusammenlaminiert werden. Die TRIS-Pufferschichten können mit wirkstoffhaltigen OTFs zusammen verabreicht werden, um einen geeigneten pH-Wert in der Mundhöhle zu erhalten. Dies kann entweder durch eine sequenzielle Verabreichung einer TRIS-Pufferschicht und eines Wirkstoff-OTFs (bzw. vice versa) erfolgen, oder die TRIS-Pufferschicht wird mit einer Wirkstoffschicht verbunden (z.B. Laminieren zu mehrschichtigen OTFs) oder die TRIS-Pufferschicht enthält selbst mindestens einen pharmazeutisch aktiven Wirkstoff. Insbesondere mit Ketamin als pharmazeutisch aktivem Wirkstoff konnten so Systeme erzeugt werden, welche vorteilhaft für eine klinische Reproduzierbarkeit (gleicht Schwankungen im Mund/Speichel-pH-Wert aus) sind und die Mundhöhle auf einen vorteilhaften pH-Wert für die Permeation puffern.

In der vorliegenden Schrift kann "umfassend" auch "bestehend aus" bedeuten.

TRIS ist eine Kurzbezeichnung für Tris(hydroxymethyl)-aminomethan (THAM), auch Tromethamin, Trometamol (INN) sowie TRIS-Puffer genannt. Chemisch handelt es sich um ein primäres Amin mit drei alkoholischen Hydroxygruppen.

TRIS wird für biochemische, molekularbiologische, mikrobiologische und pharmazeutische Zwecke als Puffersubstanz verwendet. Bei einem pKs von 8,2 (bei 20 °C) besitzt TRIS eine gute Pufferkapazität zwischen pH 7,2 bis 9,0.

Polyvinylalkohole (abgekürzt mit PVA oder PVAL, gelegentlich auch mit PVOH) sind Polymere der allgemeinen Struktur 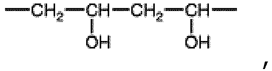 die in geringen Anteilen (etwa 2 %) auch Struktureinheiten des Typs 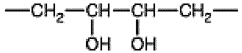 enthalten können. Sie gehören zur Gruppe der Vinylpolymere.

Handelsübliche Polyvinylalkohole, die als weiß-gelbliche Pulver oder Granulate mit Polymerisationsgraden im Bereich von etwa 500 bis 2500 (Molmassen von etwa 20000 bis 100000 g/mol) angeboten werden, haben in der Regel Hydrolysegrade von 98 bis 99 bzw. 87 bis 89 Mol-%, enthalten also noch einen Restgehalt an Acetyl-Gruppen. Charakterisiert werden die Polyvinylalkohole von Seiten der Hersteller durch Angabe des Polymerisationsgrades des Ausgangspolymeren, des Hydrolysegrades, der Verseifungszahl bzw. der Lösungsviskosität.

Gemäß der vorliegenden Erfindung sind Polyvinylalkohole mit einem mittleren Molekulargewicht von etwa 31.000 (4-88) bis etwas 205.000 (40-88) g/mol besonders geeignet.

Ferner sind gemäß der vorliegenden Erfindung Polyvinylalkohole mit einer Viskosität von von 3,4-4,6 mPas (4-88) bis 34-46 mPas (40-88) mPas in einer 40g/l wässrigen Lösung, bestimmt durch "falling ball method" (Ph.Eur. 2.2.49) , besonders geeignet, oder Mischungen aus zwei oder mehr verschiedenen PVA Typen.

Der erfindungsgemäße orale Dünnfilm zeichnet sich vorzugsweise dadurch aus, dass Polyvinylalkohol in einer Menge von 20 bis 90 Gew.-%, vorzugsweise von 40 bis 80 Gew.-% und ganz besonders bevorzugt von 50 bis 75 Gew.-%, bezogen auf das Gesamtgewicht der mindestens einen Schicht, in der mindestens einen Schicht enthalten ist.

Der erfindungsgemäße orale Dünnfilm zeichnet sich außerdem vorzugsweise dadurch aus, dass Polyvinylalkohol in einer Menge von 20 bis 50 Gew.-%, vorzugsweise von 25 bis 45 Gew.-% und ganz besonders bevorzugt von 30 bis 40 Gew.-%, bezogen auf das Gesamtgewicht der mindestens einen Schicht, in der mindestens einen Schicht enthalten ist.

Der erfindungsgemäße orale Dünnfilm zeichnet sich außerdem vorzugsweise dadurch aus, dass Polyvinylalkohol in einer Menge von 60 bis 85 Gew.-%, vorzugsweise von 65 bis 80 Gew.-% und ganz besonders bevorzugt von 65 bis 75 Gew.-%, bezogen auf das Gesamtgewicht der mindestens einen Schicht, in der mindestens einen Schicht enthalten ist.

Der erfindungsgemäße orale Dünnfilm ist ferner vorzugsweise dadurch gekennzeichnet, dass Tris(hydroxymethyl)aminomethan in einer Menge von 25 bis 55 Gew.-%, bezogen auf das Gesamtgewicht der mindestens einen Schicht, in der mindestens einen Schicht enthalten ist.

Der erfindungsgemäße orale Dünnfilm ist außerdem vorzugsweise dadurch gekennzeichnet, dass Tris(hydroxymethyl)aminomethan in einer Menge von 15 bis 45 Gew.-% oder 15 bis 40 Gew.-% .-%, bezogen auf das Gesamtgewicht der mindestens einen Schicht, in der mindestens einen Schicht enthalten ist.

Der erfindungsgemäße orale Dünnfilm ist außerdem vorzugsweise dadurch gekennzeichnet, dass Tris(hydroxymethyl)aminomethan in einer Menge von 20 bis 55 Gew.-% oder 20 bis 50 Gew.-% oder 20 bis 45 Gew.-% oder 20 bis 40 Gew.- %, bezogen auf das Gesamtgewicht der mindestens einen Schicht, in der mindestens einen Schicht enthalten ist.

Der erfindungsgemäße orale Dünnfilm ist außerdem vorzugsweise dadurch gekennzeichnet, dass Tris(hydroxymethyl)aminomethan in einer Menge von 25 bis 55 Gew.-% oder 25 bis 50 Gew.-% oder 25 bis 45 Gew.-% oder 25 bis 40 Gew.- %, bezogen auf das Gesamtgewicht der mindestens einen Schicht, in der mindestens einen Schicht enthalten ist.

Der erfindungsgemäße orale Dünnfilm ist außerdem vorzugsweise dadurch gekennzeichnet, dass Tris(hydroxymethyl)aminomethan in einer Menge von etwa 15 Gew.-% oder etwa 16, 7 Gew.-% oder etwa 25 Gew.-% oder etwa 25,5 Gew.- % oder etwa 40 Gew.-% oder etwa 50 Gew.-%, bezogen auf das Gesamtgewicht der mindestens einen Schicht, in der mindestens einen Schicht enthalten ist.

Vorzugsweise enthält der erfindungsgemäße orale Dünnfilm keine anderen Puffersubstanzen als TRIS, insbesondere keine Phosphate, Carbonate und/oder Zitronensäure.

Der erfindungsgemäße orale Dünnfilm ist ferner vorzugsweise dadurch gekennzeichnet, dass in der mindestens einen Schicht, die mindestens einen Polyvinylalkohol und Tris(hydroxymethyl)aminomethan (TRIS) enthält, keine anderen Puffersubstanzen als TRIS, insbesondere keine Phosphate, Carbonate und/oder Zitronensäure enthalten sind.

In der mindestens einen Schicht, die mindestens einen Polyvinylalkohol und Tris(hydroxymethyl)aminomethan (TRIS) enthält, sind vorzugsweise keine anderen Polymere als PVA enthalten.

Der erfindungsgemäße orale Dünnfilm ist ferner vorzugsweise dadurch gekennzeichnet, dass die mindestens eine Schicht mindestens einen pharmazeutisch aktiven Wirkstoff umfasst.

Es sind auch Ausführungsformen des erfindungsgemäßen orale Dünnfilms denkbar, in denen der orale Dünnfiln keinen pharmazeutisch aktiven Wirkstoff enthält, sondern lediglich als "Puffer-Dünnfilm" eingesetzt wird.

Der mindestens eine pharmazeutisch aktive Wirkstoff unterliegt prinzipiell keinen Beschränkungen, ist aber vorzugsweise ausgewählt aus allen pharmazeutisch aktiven Wirkstoffen, die zur oralen und/oder transmucosalen Applikation geeignet sind.

Gemäß der vorliegenden Erfindung werden unter dem pharmazeutisch aktiven Wirkstoff auch alle pharmazeutisch akzeptablen Salze und Solvate des jeweiligen pharmazeutisch aktiven Wirkstoffs subsumiert.

Bevorzugt sind Wirkstoffe ausgewählt aus der Gruppe, umfassend die Wirkstoffklassen der Analgetika, Hormone, Hypnotika, Sedativa, Antiepileptika, Weckamine, Psychoneurotropika, Neuro-Muskelblocker, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narcotika, wobei diese Gruppe nicht abschließend ist.

Besonders bevorzugt handelt es sich bei dem mindestens einen pharmazeutisch aktiven Wirkstoff um Ketamin und/oder ein pharmazeutisch akzeptables Salz oder Solvat davon, bevorzugt Ketamin·HCl.

Unter Ketamin wird hierbei (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(+)-2-(2-Chlorphenyl)-2-(methylamino)cyclohexan-1-on verstanden.

Besonders bevorzugt liegt (S)-Ketamin oder ein pharmazeutisch akzeptables Salz davon, insbesondere (S)-Ketamin·HCl, als einziges Stereoisomer von Ketamin vor, da die analgetische und anästhetische Potenz von (S)-Ketamin etwa dreifach höher als die der (R)-Form ist.

Der Wirkstoffgehalt in der mindestens einen Schicht kann innerhalb relativ weiter Grenzen variieren. Als geeignet kann ein Gehaltsbereich von 10 bis 60 Gew.-%, bezogen auf das Trockengewicht der mindestens einen Schicht, angegeben werden. In einer anderen Ausführungsform liegt der Anteil an Wirkstoff in der mindestens einen Schicht eher im Bereich von 25 bis 35 Gew.-% oder im Bereich von 25 bis 45 Gew.-% oder im Bereich von 25 bis 55 Gew.-%.

Der erfindungsgemäße orale Dünnfilm ist ferner vorzugsweise dadurch gekennzeichnet, dass die mindestens eine Schicht mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, Feuchthaltemittel, Konservierungsmittel und/oder Antioxidationsmittel, umfasst.

Jeder dieser Hilfsstoffe ist vorzugsweise jeweils in einer Menge von 0,1 bis 40 Gew.-%, vorzugsweise von 0,1 bis 30 Gew.-%, besonders bevorzugt von 0,1 bis 15 Gew.-%, ganz besonders bevorzugt von 0,1 bis 10 Gew.-% oder von 0,1 bis 5 Gew.-% bezogen auf das Gesamtgewicht der mindestens einen Schicht, in dieser Schicht enthalten.

Nach dem Trocknen bzw. dem Entfernen des Lösungsmittels ist es bevorzugt, dass der Gehalt an Restlösungsmittel in dem oralen Dünnfilm in dem Bereich von 0,2 bis 15 Gew.-%, bevorzugt in dem Bereich von 0,8 bis 7 Gew.-%, bezogen auf das Gesamtgewicht des oralen Dünnfilms, liegt

Vorzugsweise weist der orale Dünnfilm mindestens eine weitere Schicht auf, die mindestens ein Matrixpolymer und mindestens einen pharmazeutisch aktiven Wirkstoff umfasst.

In diesem Fall handelt es sich also um einen mehrschichtigen oralen Dünnfilm.

Die mindestens eine Schicht, die mindestens einen Polyvinylalkohol und Tris(hydroxymethyl)aminomethan enthält, kann mindestens einen pharmazeutisch aktiven Wirkstoff enthalten, oder in einer anderen Ausführungsform, kann diese auch keinen pharmazeutisch aktiven Wirkstoff enthalten.

Enthält die mindestens eine Schicht, die mindestens einen Polyvinylalkohol und Tris(hydroxymethyl)aminomethan enthält, keinen pharmazeutisch aktiven Wirkstoff, so ist es bevorzugt, dass mindestens eine der weiteren Schichten mindestens einen pharmazeutisch aktiven Wirkstoff enthält.

In einem derartigen Fall dient die mindestens eine Schicht, die mindestens einen Polyvinylalkohol und Tris(hydroxymethyl)aminomethan enthält, lediglich als Pufferschicht, die im Mund des Patienten die Einstellung eines gewünschten pH-Wertes bewirkt.

Ein erfindungsgemäßer orale Dünnfilm, der mindestens eine weitere Schicht aufweist, die mindestens ein Matrixpolymer und mindestens einen pharmazeutisch aktiven Wirkstoff umfasst, ist vorzugsweise dadurch gekennzeichnet, dass das mindestens eine Polymer ein wasserlösliche Polymer ist, das ausgewählt ist aus der Gruppe umfassend aus Stärke und Stärkederivate, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylate, Polyvinylpyrrolidone, Vinvlpyrrolidon/Vinylacetat-Copolymere, Polyvinylalkohole, Polyethylenoxidpolymere, Polyacrylamide, Polyethylenglycole, Gelatine, Collagen, Alginate, Pektin, Pullulan, Traganth, Chitosan, Alginsäure, Arabinogalaktan, Galaktomannan, Agar, Agarose, Carrageen und natürlichen Gummen.

Der mindestens eine pharmazeutisch aktive Wirkstoff unterliegt prinzipiell keiner Beschränkung, sondern ist vorzugsweise ausgewählt unter allen pharmazeutisch aktiven Wirkstoffen, die zur oralen und/oder transmucosalen Applikation geeignet sind.

Alle aufgeführten Wirkstoffe umfassen zudem pharmazeutisch akzeptable Salze und/oder Solvate davon.

Ein erfindungsgemäßer orale Dünnfilm, der mindestens eine weitere Schicht aufweist, die mindestens ein Matrixpolymer und mindestens einen pharmazeutisch aktiven Wirkstoff umfasst, ist vorzugsweise dadurch gekennzeichnet, dass der mindestens eine pharmazeutisch aktive Wirkstoff ausgewählt ist aus der Gruppe, umfassend die Wirkstoffklassen der Analgetika, Hormone, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narcotika, wobei der mindestens eine pharmazeutisch aktive Wirkstoff vorzugsweise Ketamin, besonders bevorzugt (S)-Ketamin, umfasst.

In einer Ausführungsform ist der erfindungsgemäße mehrschichtige orale Dünnfilm dadurch gekennzeichnet, dass die mindestens eine Schicht, die mindestens einen Polyvinylalkohol und Tris(hydroxymethyl)aminomethan enthält, und/oder die mindestens eine weitere Schicht, die mindestens ein Matrixpolymer und mindestens einen pharmazeutisch aktiven Wirkstoff umfasst, direkt aufeinander laminiert sind.

In einer anderen Ausführungsform ist der erfindungsgemäße mehrschichtige orale Dünnfilm dadurch gekennzeichnet, dass die mindestens eine Schicht, die mindestens einen Polyvinylalkohol und Tris(hydroxymethyl)aminomethan enthält, und/oder die mindestens eine weitere Schicht, die mindestens ein Matrixpolymer und mindestens einen pharmazeutisch aktiven Wirkstoff oder mindestens einen Geschmacksstoff umfasst, durch eine dazwischenliegende Klebeschicht miteinander verbunden sind.

Unter Klebeschicht wird eine Schicht verstanden, die als Klebstoff, wie in der DIN EN 923:2016-03 definiert, wirken kann. Eine nicht-klebende Schicht kann demnach nicht als Klebstoff wie vorstehend definiert wirken.

Als Klebeschichten eignen sich insbesondere wasserlösliche Klebeschichten, wie diese in der DE 10 2014 127 452 A1 beschrieben werden, deren diesbezüglicher Inhalt hiermit vollständig einbezogen wird.

Solche Klebeschichten umfassen mindestens ein wasserlösliches Polymer und mindestens einen Weichmacher, wobei das mindestens eine wasserlösliche Polymer in der mindestens einen wasserlöslichen Klebeschicht vorzugsweise Schellack, ein Vinylpyrrolidon-/Vinylacetat-Copolymer, ein Polyvinylcaprolactam- /Polyvinylacetat-/Polyethyleneglycol-Copolymer, Hydroxypropylcellulose oder Hydroxypropylmethylcellulose und/oder Polyvinylpyrrolidon umfasst, und wobei der mindestens eine Weichmacher in der mindestens einen wasserlöslichen Klebeschicht vorzugsweise Glycerin, Polyethylenglycol, insbesondere Polyethylenglycol 200, und/oder Tributylcitrat umfasst.

Das Gewichtsverhältnis des mindestens einen wasserlöslichen Polymers zu dem mindestens einen Weichmacher in der mindestens einen Klebeschicht beträgt vorzugsweise etwa 90 bis 50 zu etwa 10 bis 50, vorzugsweise etwa 85 bis 50 zu etwa 15 zu 50.

Eine solche Klebeschicht, die mindestens ein wasserlösliches Polymer und mindestens einen Weichmacher enthält, kann als dazwischenliegende wasserlösliche Klebeschicht zwei weitere Schichten, die an sich nicht klebrig sind, fest miteinander verkleben und somit den Aufbau von mehrschichtigen oralen Filmen ohne mehrfacher Übereinanderbeschichtung ermöglichen, was erhöhte Trockenzeiten und erhöhten Temperaturstress für die enthaltenen Wirk-und Hilfsstoffe bedeuten würde.

In einer anderen Ausführungsform ist der erfindungsgemäße mehrschichtige orale Dünnfilm dadurch gekennzeichnet, dass die mindestens eine Schicht, die mindestens einen Polyvinylalkohol und Tris(hydroxymethyl)aminomethan enthält, und/oder die mindestens eine weitere Schicht, die mindestens ein Matrixpolymer und mindestens einen pharmazeutisch aktiven Wirkstoff oder mindestens einen Geschmacksstoff umfasst, durch eine dazwischenliegende Trennschicht miteinander verbunden sind.

Als Trennschicht wird vorzugsweise eine Schicht, die mindestens ein Polyethylenglycol umfasst, eingesetzt.

Polyethylenglycole (PEG) sind Verbindungen der allgemeinen Formel:

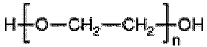

Höhermolekulare feste Polyethylenglycole (Schmelztemperatur etwa 65 °C) werden häufig auch als Polyethylenoxide oder auch Polyoxyethylene (Kurzzeichen PEO oder seltener PEOX) oder Polywachse bezeichnet. In der vorliegenden Schrift werde die Begriffe "Polyethylenglycol", "Polyethylenoxid" und "PolyOx"gleichwertig verwendet.

Das mindestens eine Polyethylenglycol weist vorzugsweise ein mittleres Molekulargewicht von mindestens 20.000 g/mol bis 7.000.000 g/mol, vorzugsweise von 40.000 g/mol bis 500.000 g/mol, besonders bevorzugt von 95.000 g/mol bis 105.000 g/mol, insbesondere von etwa 100.000 g/mol auf.

Das Molekulargewicht wird aus den nachfolgend beschriebenen Rheologiemessungen abgeleitet.

Das mindestens eine Polyethylenglycol weist vorzugsweise eine Viskosität von 30 mPas bis 50 mPas, gemessen bei 25°C, auf.

Die angegebenen Viskositäten beziehen sich jeweils auf eine 5 Gew.-%-ige Lösung des Polyethylenglycols in Wasser und werden auf einem Brookfield Viskosimeter, Model RVF, mit Spindel No. 1 bei 50 rpm und bei einer Temperatur von 25°C gemessen.

Besonders bevorzugt wird ein Polyethylenglycol eingesetzt, das unter dem Handelsnamen POLYOX WSR N-10 (Dow Chemical) bekannt ist.

Das mindestens eine Polyethylenglycol ist vorzugsweise in einer Menge von 60 bis 100 Gew.-%, vorzugsweise in einer Menge von 80 bis 100 Gew.-%, bezogen auf das Gesamtgewicht der mindestens einen Trennschicht, in der mindestens einen Trennschicht enthalten.

Die Trennschicht enthält vorzugsweise mindestens einen Weichmacher, vorzugsweise Glycerol, vorzugsweise in einer Menge von 0,5 bis 5 Gew.-%, besonders bevorzugt in einer Menge von 2 bis 2,5 Gew.-%, bezogen auf das Gesamtgewicht der mindestens einen Trennschicht.

Der Einsatz einer solchen Trennschicht hat u.a. den Vorteil, dass sich Polyethylenglycol-Filme aufgrund ihrer glatten Oberfläche, ihrer niedrigen Schmelz- bzw. Glasübergangstemperatur, ihrer unbedenklicher Toxizität und ihrer Wasserlöslichkeit gut als Trennschichten/Verbindungsschichten/Klebeschichten in mehrschichtigen oralen Dünnfilmen eignen. Bei Erwärmen und/oder bei hohem Druck eignen sich Polyethylenglycol-Klebeschichten insbesondere dazu zwei weitere Schichten miteinander zu verbinden. Dabei können beispielsweise eine wirkstoffhaltige Schicht und eine pH-regulierende Puffer-Schicht miteinander verbunden werden.

Des Weiteren eignet sich die Polyethylenglycol-Schicht als Sperrschicht, welche die Migration von Wirkstoffen oder Hilfsstoffen (z.B. Puffersalzen) zwischen den einzelnen Schichten verhindert bzw. minimiert.

Die Trennschicht benötigt zudem keine oder nur geringe Mengen Hilfsstoffe wie Weichmacher (z.B. 2-3% Glycerin) und reduziert somit das Risiko einer Migration der Klebeschichtbestandteile in die anderen Schichten des oralen Dünnfilms.

Der erfindungsgemäße mehrschichtige oralen Dünnfilm ist bezüglich seines Aufbaus keinen Beschränkungen unterworfen.

So sind Ausführungsform dankbar in denen der mehrschichtige oralen Dünnfilm eine TRIS-Pufferschicht, die keinen pharmazeutisch aktiven Wirkstoff enthält, und eine weitere Schicht, die mindestens ein Matrixpolymer und mindestens einen pharmazeutisch aktiven Wirkstoff enthält, umfasst.

Diese beiden Schichten können direkt aufeinander laminiert werden oder mit einer dazwischenliegenden Klebeschicht oder Trennschicht, vorzugsweise wie oben definiert, verbunden werden.

Es sind auch Ausführungsformen denkbar in denen der mehrschichtige orale Dünnfilm aus einer TRIS-Pufferschicht, die keinen pharmazeutisch aktiven Wirkstoff enthält, und einer weiteren Schicht, die mindestens ein Matrixpolymer und mindestens einen pharmazeutisch aktiven Wirkstoff enthält, besteht.

Diese beiden Schichten können direkt aufeinander laminiert werden oder mit einer dazwischenliegenden Klebeschicht, vorzugsweise wie oben definiert, verbunden werden.

Es sind auch Ausführungsformen denkbar in denen der mehrschichtige orale Dünnfilm eine TRIS-Pufferschicht, die keinen pharmazeutisch aktiven Wirkstoff enthält, und einer weiteren Schicht, die mindestens ein Matrixpolymer und mindestens einen pharmazeutisch aktiven Wirkstoff enthält, wobei diese beiden Schichten durch eine Klebeschicht oder eine Trennschicht, wie oben definiert, verbunden sind, umfasst.

Es sind auch Ausführungsform denkbar in denen der mehrschichtige orale Dünnfilm aus einer TRIS-Pufferschicht, die keinen pharmazeutisch aktiven Wirkstoff enthält, und einer weiteren Schicht, die mindestens ein Matrixpolymer und mindestens einen pharmazeutisch aktiven Wirkstoff enthält, wobei diese beiden Schichten durch eine Klebeschicht oder eine Trennschicht, wie oben definiert verbunden sind, besteht.

Es sind weitere mehrschichtige orale Dünnfilm denkbar, die beispielsweise folgenden Schichtaufbau aufweisen: TRIS-Pufferschicht - Schicht, die mindestens ein Matrixpolymer und mindestens einen pharmazeutisch aktiven Wirkstoff enthält, - TRIS, Pufferschicht.

In dieser Ausführungsform bilden die beiden TRIS-Pufferschichten Außenseiten des mehrschichtigen oralen Dünnfilms.

Diese drei Schichten können entweder direkt aufeinander laminiert werden oder mit einer dazwischenliegenden Klebeschicht, vorzugsweise wie oben definiert, verbunden werden.

Prinzipiell lässt sich so ein Baukastensystem bereitstellen, und zwar mit beliebigen Anordnungen von TRIS-Pufferschichten und weiteren Schichten.

In all den vorstehend beschriebenen Ausführungsformen enthält die TRIS-Pufferschicht vorzugsweise keinen pharmazeutisch aktiven Wirkstoff. Es sind jedoch auch Ausführungsformen denkbar in denen auch in dem mehrschichtigen Dünnfilm in den TRIS-Pufferschichten jeweils mindestens ein pharmazeutischer aktiver Wirkstoff enthalten ist.

Der erfindungsgemäße orale Dünnfilm ist ferner vorzugsweise dadurch gekennzeichnet, dass die mindestens eine Schicht, die mindestens einen Polyvinylalkohol und Tris(hydroxymethyl)aminomethan enthält, und/oder die mindestens eine weitere Schicht, die mindestens ein Matrixpolymer und mindestens einen pharmazeutisch aktiven Wirkstoff umfasst, in Form eines Hohlräume aufweisenden verfestigten Schaums vorliegt (vorliegen).

Durch die Hohlräume und die damit verbundene größere Oberfläche der Filme wird insbesondere der Zutritt von Wasser bzw. Speichel oder anderen Körperflüssigkeiten in das Innere der Darreichungsform erleichtert und somit die Auflösung der Darreichungsform und die Wirkstofffreisetzung beschleunigt.

Bei einem schnell resorbierenden Wirkstoff kann zudem durch die schnelle Auflösung der Matrixschicht die transmucosale Resorption verbessert werden. Zum anderen ist die Wandstärke der genannten Hohlräume vorzugsweise gering, da diese beispielsweise verfestigte Blasen darstellen, so dass eine schnelle Auflösung oder Zerstörung dieser Hohlräume stattfindet.

Ein weiterer Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der erfindungsgemäße orale Dünnfilm dadurch gekennzeichnet, dass die Hohlräume voneinander isoliert sind und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium, Argon oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 50 bis 80 %, bezogen auf das Gesamtvolumen der jeweiligen Schicht. Auf diese Weise wird der beabsichtigte Effekt, die Lösung der wirkstoffhaltigen Matrixschicht zu beschleunigen, in günstiger Weise beeinflusst.

Ferner können dem Matrix-Polymer bzw. der Polymermatrix zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften der erfindungsgemäßen Darreichungsform beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 1 bis 350 µm liegen. Besonders bevorzugt liegt der Durchmesser im Bereich von 30 und 200 µm.

Der erfindungsgemäße orale Dünnfilm besitzt vorzugsweise eine Fläche von 0,5 cm² bis etwa 10 cm², besonders bevorzugt von 1,5 cm² bis etwa 9 cm².

Der erfindungsgemäße orale Dünnfilm ist vorzugsweise dadurch gekennzeichnet, dass das Flächengewicht des oralen Dünnfilms 10 bis 500 g/m², vorzugsweise 100 bis 400 g/m², beträgt.

Das Flächengewicht der TRIS-Pufferschicht und jeder weiteren eventuell vorhandenen Schicht beträgt jeweils vorzugsweise mindestens 10 g/m², bevorzugter mindestens 20 g/m² oder mindestens 30 g/m² oder am meisten bevorzugt mindestens 50 g/m² oder von weniger als oder gleich 400 g/m², bevorzugter weniger als oder gleich 350 g/m² oder weniger als oder gleich 300 g/m² oder am meisten bevorzugt weniger als 250 g/m². Vorzugsweise beträgt das Flächengewicht 10 bis 400 g/m², bevorzugter 20 bis 350 g/m² oder 30 bis 300 g/m² am meisten bevorzugt 50 bis 250 g/m².

Bevorzugt weist die TRIS-Pufferschicht und jede weitere eventuell vorhandene Schicht jeweils eine Schichtdicke von vorzugsweise 10 µm bis 500 µm, besonders bevorzugt von 20 µm bis 300 µm, auf.

Liegt die TRIS-Pufferschicht in Form eines Schaumes vor, so weist die TRIS-Pufferschicht eine Schichtdicke von vorzugsweise 10 µm bis 3000 µm, besonders bevorzugt von 90 µm bis 2000 µm, auf.

Der erfindungsgemäße orale Dünnfilm ist ferner vorzugsweise dadurch gekennzeichnet, dass der orale Dünnfilm im Mund eines Patienten, vorzugsweise eines Menschen, einen pH-Wert (bei 37°C) von 6 bis 9, vorzugsweise von 6 bis 8, erzeugt.

Der erfindungsgemäße orale Dünnfilm ist ferner vorzugsweise dadurch gekennzeichnet, dass der orale Dünnfilm in 2 mL Humanspeichel, künstlichem Speichel, PBS-Puffer oder Wasser einen pH-Wert (bei 37°C) von 6 bis 8 erzeugt.

Der erfindungsgemäße orale Dünnfilm ist ferner vorzugsweise dadurch gekennzeichnet, dass der orale Dünnfilm eine TRIS-Pufferschicht und eine weitere Schicht, die S-Ketamin HCI enthält, umfasst und in 2 mL Humanspeichel, künstlichem Speichel, PBS-Puffer oder Wasser einen pH-Wert (bei 37°C) von 6 bis 8 erzeugt.

Der erfindungsgemäße orale Dünnfilm ist ferner vorzugsweise dadurch gekennzeichnet, dass der orale Dünnfilm eine TRIS-Pufferschicht und eine weitere Schicht, die 16,3 mg S-Ketamin HCI enthält, umfasst und in 2 mL Humanspeichel, künstlichem Speichel, PBS-Puffer oder Wasser einen pH-Wert (bei 37°C) von 6 bis 8 erzeugt.

Der erfindungsgemäße orale Dünnfilm ist ferner vorzugsweise dadurch gekennzeichnet, dass der orale Dünnfilm eine TRIS-Pufferschicht und eine weitere Schicht, die 32,2 mg S-Ketamin HCI enthält, umfasst und in 2 mL Humanspeichel, künstlichem Speichel, PBS-Puffer oder Wasser einen pH-Wert (bei 37°C) von 6 bis 8 erzeugt.

Die vorliegende Erfindung betrifft auch einen Kit, umfassend mindestens einen ersten oralen Dünnfilm, der mindestens eine Schicht, die mindestens einen Polyvinylalkohol und Tris(hydroxymethyl)aminomethan umfasst, und mindestens einen zweiten oralen Dünnfilm, der mindestens ein Matrixpolymer und mindestens einen pharmazeutisch aktiven Wirkstoff umfasst, wobei der erste und zweite orale Dünnfilm vorzugsweise unterschiedlich ist. Vorzugsweise enthält in diesem Kit der erste orale Dünnfilm keinen pharmazeutisch aktiven Wirkstoff.

Obige Ausführungen für die TRIS-Pufferschicht und die weitere Schicht gelten analog für den ersten und zweiten oralen Dünnfilm, die in dem erfindungsgemäßen Kit vorhanden sind.

Der erfindungsgemäße orale Dünnfilm kann gemäß dem Fachmann bekannten Verfahren hergestellt werden.

Bekannte Herstellungsverfahren umfassen das Bereitstellen einer Lösung, umfassend PVA und TRIS, das anschließende Ausstreichen und Trocknen dieser Lösung, um einen Film zu erhalten.

Soll ein mehrschichtige orale Dünnfilm hergestellt werden, so umfasst ein bekanntes Herstellungsverfahren das Bereitstellen einer ersten Lösung, umfassend PVA und TRIS, das anschließende Ausstreichen und Trocknen dieser Lösung, um einen Film zu erhalten und das Bereitstellen einer zweiten Lösung, umfassend das mindestens eine Matrixpolymer und mindestens einen pharmazeutisch aktiven Wirkstoff, um einen zweiten Film zu erhalten.

Das Zusammenfügen dieser beiden Filme kann prinzipiell durch dem Fachmann geläufige Methoden erfolgen. So kann beispielsweise auf einen ersten Film mittels Beschichtung ein weiterer Film aufgebracht werden, wobei es unerheblich ist, welcher Film auf welchen Film beschichtet wird. Ferner können die beiden Schichten durch eine Klebeschicht, wie vorstehend beschrieben, miteinander verbunden werden.

Die vorliegende Erfindung betrifft ferner einen oralen Dünnfilm, erhältlich nach dem vorstehend beschriebenen Verfahren.

Außerdem betrifft die vorliegende Erfindung einen oralen Dünnfilm, wie vorstehend beschrieben, erhältlich nach dem vorstehend beschriebenen Verfahren oder den vorstehend beschriebenen Kit als Arzneimittel.

Die vorliegende Erfindung betrifft zudem einen oralen Dünnfilm, wie vorstehend beschrieben, erhältlich nach dem vorstehend beschriebenen Verfahren oder der vorstehend beschriebene Kit, wobei Ketamin, vorzugsweise S-Ketamin (ggf. als pharmazeutisch akzeptables Salz oder Ionenpaar), als pharmazeutisch aktiver Wirkstoff eingesetzt wird, als Arzneimittel zur Verwendung in der Behandlung von Schmerz und/oder Depressionen, insbesondere zur Reduzierung des Suizidrisikos und/oder zur Verwendung als Allgemeinanästhetikum, vorzugsweise zur Einleitung und Durchführung einer Vollnarkose oder als Ergänzung bei Regionalanästhesien und/oder als Analgetikum.

Die vorliegende Erfindung wird nachfolgend in einigen nicht beschränkenden Beispielen näher erläutert.

### Beispiele

### Beispiel 1:

Einfluss des pH-Wertes auf die Permeation von (S)-Ketamin HCI
Saurer Puffer:
   80 g/l Na₂HPO₄x2H₂O gelöst in Wasser; pH Einstellung mit wässriger 1M HCI Lösung auf pH=4,99 bei 22°C.
Neutral Buffer:
   80 g/l Na₂HPO₄x2H₂O gelöst in Wasser; pH Einstellung mit wässriger 1M HCI Lösung auf pH=7,46 bei 22°C.
Basischer Puffer:
   80 g/l Na₂HPO₄x2H₂O gelöst in Wasser; pH Einstellung mit wässriger 1M HCI Lösung auf pH=8,33 bei 22°C.

Die pH-Werte dieser Versuchsreihe sind in Tabelle 1 zusammengefasst.

**Tabelle 1:**

| | Puffer pH bei 22°C | Puffer pH bei 37°C | Puffer + (S)-Ketamin HCI pH bei 22°C |
|---|---|---|---|
| Saurer Puffer | 4,99 | 5,09 | 4,92 |
| Neutraler Puffer | 7,46 | 7,48 | 6,64 |
| Basischer Puffer | 8,33 | 7,91 | 7,81 |

Die permeierte Menge an (S)-Ketamin wurde durch in-vitro-Experimente gemäß der OECD-Richtlinie (verabschiedet am 13. April 2004) unter Verwendung der Schweineschleimhaut (Mucosa oesophagus) und einer "Franz-Diffussionszelle" bestimmt. Mit einem Dermatom wurde die Schleimhaut bis zu einer Dicke von 400 µm und 1,145 cm² präpariert, bei intakter Barrierefunktion für alle transmukosalen therapeutischen Systeme.

Als Akzeptormedium dienten 4 mL PBS-Puffer mit einem pH-Wert von 7,4 bei 37°C.

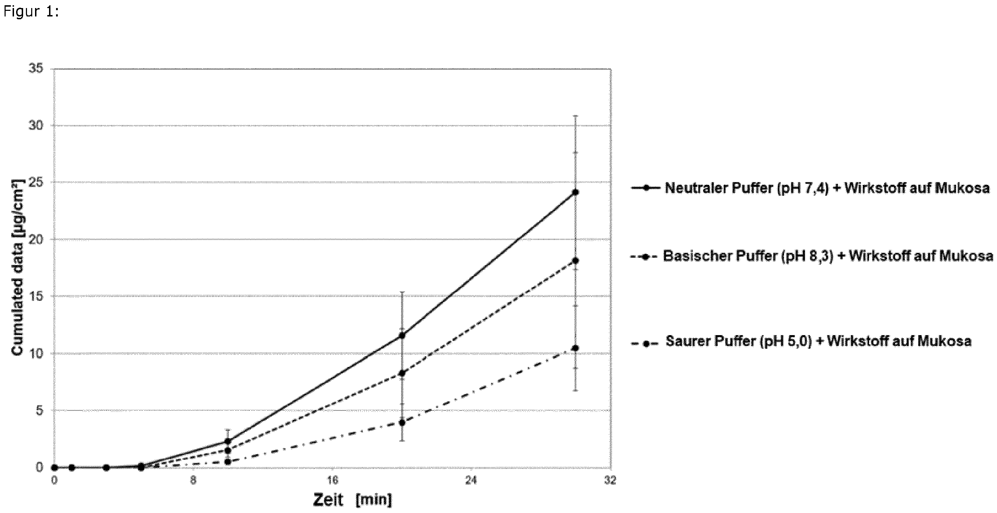

2,77 mg (S)-Ketamin HCI wurde auf die Schleimhaut aufgetragen, und die Schleimhaut mit dem Wirkstoff wurde auf der Oberseite mit der jeweiligen Pufferlösung in Kontakt gebracht. Die Unterseite der Schleimhaut stand mit dem Akzeptormedium in Kontakt. Die permeierte Menge an S-Ketamin im Rezeptormedium bei einer Temperatur von 37 ± 1°C wurde gemessen. Die Ergebnisse sind in Figur 1 dargestellt.

Aus den erhaltenen Permeationsergebnissen kann geschlossen werden, dass die Permeation von S-Ketamin in neutralem Puffer, gefolgt von basischem Puffer, am besten ist. Die niedrigste Permeationsrate wurde mit saurem Puffer beobachtet.

### Beispiel 2:

Einfluss des pH-Wertes auf die Permeation von (S)-Ketamin HCI aus einem oralen Dünnfilm.

Ein oraler Dünnfilm der Zusammensetzung gemäß Tabelle 2 wurde hergestellt.

**Tabelle 2:**

| **Material** | **Formulierung 1 [Gew.-%]** |
|---|---|
| PVA 4-88 | 39.1 |
| TRIS | -- |
| Saccharin Na | 1,0 |
| Sucralose | 2,0 |
| Cherry Flavor M55394 | 3,0 |
| Glycerol | 4,5 |
| S-Ketamin HCI | 50,0 |
| FD&C Red No. 40 | 0,4 |
| Kollidon VA 64 | -- |
| Lösungsmittel | Aqua Purificata |
| Flächengewicht (incl. Restfeuchtigkeit): | 199 g/m² |

| | |
|---|---|
| PVA 4-88: Polyvinylalkohol Geschmacksstoff: MANE Cherry Flavor M55394 Farbstoff: FD&C Red No. 40 | |

Der orale Dünnfilm mit der Formulierung gemäß Tabelle 2 wird mittels dem Fachmann bekannte Techniken der Masseherstellung, z.B. durch Rühren/Mischen der enthaltenen Komponenten mittels Rührmotor und geeigneter Rührwerkzeuge hergestellt.

In die so erhaltene Masse wird durch Rühren Gas eingeschlagen, z.B. mittels einer Schaumaufschlagmaschine.

Die schaumige Masse wird durch geeignete Geräte (Walzenauftragswerk, Rakel, Streichkasten, etc) in einer konstanten Schichtdicke auf einen Beschichtungsträger beschichtet.

Als Beschichtungsträger können alle temperaturstabilen bahnförmigen Materialien dienen, von denen sich der trockene Film wieder entfernen lässt. Dies kann durch die Materialauswahl des Beschichtungsträgers gewährleistet werden (unterschiedliche Oberflächenspannungen zwischen schaumförmiger Masse und Substrat), oder durch geeignete dehäsive Beschichtungen des Beschichtungsträgers mit z.B. Silikonen oder Fluorpolymeren.

Das oder die enthaltenen Prozesslösemittel, im Regelfall Wasser oder Mischungen aus Wasser und organischen, wassermischbaren Lösemitteln, werden durch Trocknen entfernt. Aus der so erhaltenen, festen Schaumschicht lassen sich die oralen Dünnfilme durch Schneiden oder Stanzen in der entsprechenden Größe konfektionieren.
Saurer Puffer:
   80 g/l Na₂HPO₄x2H₂O gelöst in Wasser; pH Einstellung mit wässriger 1M HCI Lösung auf pH=4,99 bei 22°C.
Neutral Buffer:
   80 g/l Na₂HPO₄x2H₂O gelöst in Wasser; pH Einstellung mit wässriger 1M HCI Lösung auf pH=7,46 bei 22°C.
Basischer Puffer:
   80 g/l Na₂HPO₄x2H₂O gelöst in Wasser; pH Einstellung mit wässriger 1M HCI Lösung auf pH=8,33 bei 22°C.

Die pH-Werte dieser Versuchsreihe sind in Tabelle 3 zusammengefasst.

**Tabelle 3:**

| | Puffer pH bei 22°C | Puffer pH bei 27°C | Puffer + OTF pH bei 22°C | Puffer + OTF pH bei 37°C |
|---|---|---|---|---|
| Saurer Puffer | 4,99 | 5,09 | 4,92 | 5,05 |
| Neutraler Puffer | 7,46 | 7,48 | 6,88 | 6,72 |
| Basischer Puffer | 8,33 | 7,91 | 7,91 | 7,50 |

0,287 cm² OTF (entspricht 2,77 mg (S)-Ketamin) der S-Ketamin HCI-Formulierung 1 wurde auf die Schleimhaut gelegt und 70 µL Pufferlösung hinzugefügt.

Die Permeation wurde ähnlich wie in Beispiel 1 gemessen.

Die permeierte Menge an (S)-Ketamin wurde durch in-vitro-Experimente gemäß der OECD-Richtlinie (verabschiedet am 13. April 2004) unter Verwendung der Schweineschleimhaut (Mucosa oesophagus) und einer "Franz-Diffussionszelle" bestimmt. Mit einem Dermatom wurde die Schleimhaut bis zu einer Dicke von 400 µm präpariert, bei intakter Barrierefunktion für alle transmukosalen therapeutischen Systeme.

Aus den OTFs wurden Stanzungen mit einer Fläche von 0,287 cm² ausgestanzt, auf die Schleimhaut appliziert und die Schleimhaut mit den OTFs auf der Oberseite mit dem jeweiligen Puffer in Kontakt gebracht (die Unterseite stand mit dem Akzeptormedium in Kontakt und die Oberseite wurde auf eine Schleimhautfläche von 1,145 cm² abgeschottet).

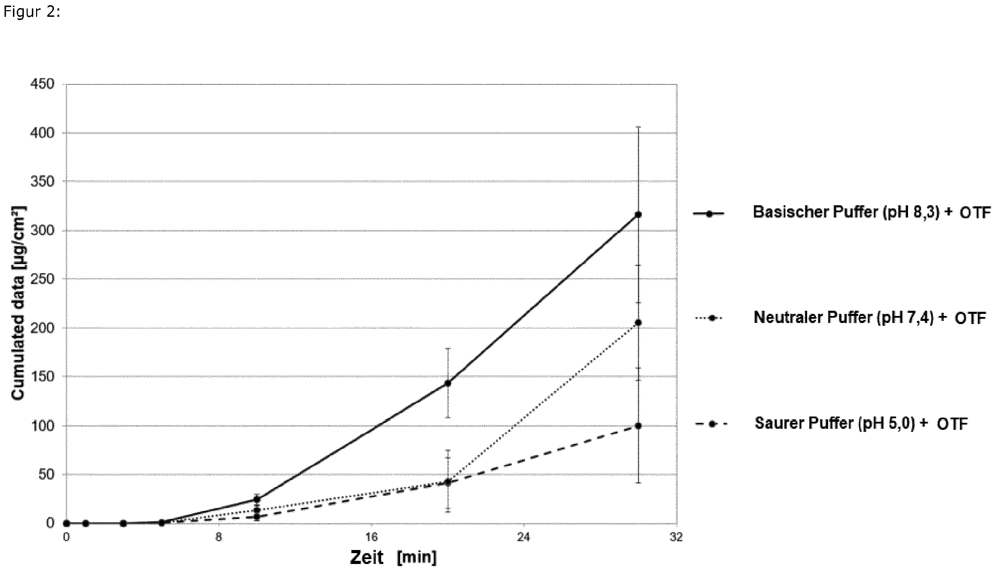

Die permeierte Menge an S-Ketamin im Rezeptormedium (Phosphatpufferlösung pH 7,4) bei einer Temperatur von 37 ± 1°C wurde gemessen. Die Ergebnisse sind in Figur 2 dargestellt.

Die in vitro-Experimente mit den verschiedenen Puffern als Donormedium zeigten ein Permeationsverhalten von S-Ketamin, das vom pH-Wert des verwendeten Puffers abhängig war. Der pH-Wert des Spendermediums einschließlich Wirkstoff ist in Tabelle 2 (Puffer + OTF) dargestellt.

Aus den erhaltenen Permeationsergebnissen kann geschlossen werden, dass die Permeation von S-Ketamin in basischem Puffer am besten ist, gefolgt von neutralem Puffer. Die niedrigste Permeationsrate wurde mit saurem Puffer beobachtet.

### Beispiel 3:

**Tabelle 4:**

| | Formulierungen (Mengen in Gew.-%) | | | | | | |
|---|---|---|---|---|---|---|---|
| Material | 2 | 3 | 4 | 5 | 6 | 7 | 8 |
| PVA 4-88 | 36,15 | 69,30 | 59,50 | 64,00 | 74,50 | 39,50 | 39,0 |
| PVA 40-88 | 36,15 | -- | -- | --- | -- | -- | -- |
| TRIS | 20,00 | 20,00 | 30,00 | 25,50 | 15,00 | 50,00 | 50,00 |
| Saccharin Na | 1,00 | 1,00 | 1,00 | 1,00 | 1,00 | 1,00 | 1,00 |
| Sucralose | 2,00 | 2,00 | 2,00 | 2,00 | 2,00 | 2,00 | 2,00 |
| Geschmacksstoff | - | 3,0 | 3,00 | 3,00 | 3,00 | 3,00 | 3,00 |
| Glycerol | 4,50 | 4,50 | 4,50 | 4,50 | 4,50 | 4,50 | 4,50 |
| Farbstoff | 0,20 | 0,20 | -- | -- | -- | -- | -- |
| Aerosil | -- | -- | -- | -- | -- | -- | 0,5 |
| Prozesslösemittel: | Wasser | Wasser | Wasser | Wasser | Wasser | Wasser | Wasser |
| Gas für Schäumung | Stickstoff | Luft | Luft | Luft | Luft | Luft | Luft |
| pH | 3,34 cm² OTF in 2 mL Wasser pH=9,0 (36 °C) | --- | --- | 3,34 cm² OTF in 2 mL Wasser | --- | 3,34 cm² OTF in 2 mL Wasser pH=9,61 (22 °C) | --- |
| | | | | pH=9,65 (21 °C) | | 3,34 cm² OTF in 2 mL Glandosane pH=9,01 (21 °C) | |

| | | | | | | | |
|---|---|---|---|---|---|---|---|
| PVA 4-88: Polyvinylalkohol PVA 40-88: Polyvinylalkohol Geschmacksstoff: MANE Cherry Flavor M55394 Farbstoff: FD&C Red No. 40 Aerosil 200: Hydrophile pyrogene Kieselsäure mit einer spezifischen Oberfläche von 200 m²/g (EVONIK) Glandosane: künstlicher Speichel | | | | | | | |

Orale Dünnfilme mit den Formulierungen gemäß Tabelle 3 wurden gemäß Beispiel 2 hergestellt.

Die oralen Dünnfilme mit den Formulierungen gemäß Tabelle 4 zeichnen sich durch große Flexibilität und angenehme Haptik ("Mundgefühl") aus. Desweiteren lösen sich die so hergestellten oralen Dünnfilme, trotz des u.U. großen Flächengewichtes und der großen Dicke sehr schnell im Mund auf. Bedingt durch die dünnen Schichten der Wände der Gasblasen ist eine sehr schonende Trocknung möglich (z.B. 20 Minuten bei 70°C für 200 g/m²).

### Beispiel 4:

**Tabelle 5:**

| Material | Formulierung 9 [Gew.-%] |
|---|---|
| PVA 4-88 | 39,5 |
| TRIS | 16,7 |
| Saccharin Na | 1,0 |
| Sucralose | 2,0 |
| Geschmacksstoff | 3,0 |
| Glycerol | 4,50 |
| S-Ketamin | 33,3 |
| Gas: | Luft (alternativ Stickstoff) |
| Prozesslösemittel | Wasser |
| Flächengewicht (incl. Restfeuchtigkeit) | 2x 141 g/m² zusammenlaminiert |
| pH | 3.34 cm² OTF in 2 mL Aqua purificata: |
| | pH=7.5 (22 °C) |
| | pH=7.2 (33 °C) |
| | 3.34 cm² OTF in 2 mL Humanspeichel: |
| | pH=7,45 (22°C) |
| | pH=7.28 (33,5 °C) |

| | |
|---|---|
| PVA 4-88: Polyvinylalkohol Geschmacksstoff: MANE Cherry Flavor M55394 | |

Die Herstellung von einem oralen Dünnfilm der Formulierung gemäß Tabelle 5 erfolgt durch dem Fachmann bekannte Techniken der Masseherstellung, z.B. durch Rühren/Mischen der enthaltenen Komponenten mittels Rührmotor und geeigneter Rührwerkzeuge.

In die so erhaltene Masse wird durch Rühren Gas eingeschlagen, z.B. mittels einer Schaumaufschlagmaschine. Die schaumige Masse wird durch geeignete Geräte (Walzenauftragswerk, Rakel, Streichkasten, etc) in einer konstanten Schichtdicke auf einen Beschichtungsträger beschichtet.

Als Beschichtungsträger können alle temperaturstabilen bahnförmigen Materialien dienen, von denen sich der trockene Film wieder entfernen lässt. Dies kann durch die Materialauswahl des Beschichtungsträgers gewährleistet werden (unterschiedliche Oberflächenspannungen zwischen schaumförmiger Masse und Substrat), oder durch geeignete dehäsive Beschichtungen des Beschichtungsträgers mit z.B. Silikonen oder Fluorpolymeren. Das oder die enthaltenen Prozesslösemittel, im Regelfall Wasser oder Mischungen aus Wasser und organischen, wassermischbaren Lösemitteln, werden durch Trocknen entfernt.

Anschließend wurden die beiden Laminate auf 70°C erhitzt und aufeinander laminiert. Aus dem so erhaltenen zweischichtigen Laminat lassen sich die oralen Dünnfilme durch Schneiden oder Stanzen in der entsprechenden Größe konfektionieren.

Dieser zweischichtige orale Dünnfilm zeichnet sich durch seine hohe Schichtdicke und Beladung aus. Der Vorgang kann beliebig oft wiederholt werden, und somit auch mehrschichtige Laminate erhalten werden (z.B. drei Laminate zusammenlaminieren, oder vier Laminate), bzw. können auch mehrere Laminate in einem Schritt zusammenlaminiert werden (z.B. drei Laminate auf 70 °C erhitzen und zusammenlaminieren).

### Beispiel 5

**Tabelle 6:**

| | Formulierung (Menge in Gew.-%) | | |
|---|---|---|---|
| Material | Formulierung 10 | Formulierung 11 | Formulierung 9 |
| PVA 4-88 | 37,5 | 39,5 | 39,5 |
| TRIS | 2,0 | 25,0 | 16,7 |
| Saccharin Na | 1,0 | 1,0 | 1,0 |
| Sucralose | 2,0 | 2,0 | 2,0 |
| Geschmacksstoff | 3,0 | 3,0 | 3,0 |
| Glycerol | 4,5 | 4,5 | 4,5 |
| S-Ketamin HCI | 50,0 | 25,0 | 33,3 |
| Gas: | Luft (alternativ Stickstoff) | Luft (alternativ Stickstoff) | Luft (alternativ Stickstoff) |
| Prozesslösemittel | Wasser | Wasser | Wasser |
| Flächengewicht (incl. Restfeuchtigkeit) | 223 g/m² | 175 g/m² | 2x141 g/m² zusammenlaminiert |
| pH | 3.34 cm² OTF in 2 mL Aqua purificata pH=6.0 (22°C) | 3.34 cm² OTF in 2 mL Aqua purificata pH=8.26 (24 °C) | 3.34 cm² OTF in 2 mL Aqua purificata: pH=7.5 (22 °C) |
| | 3.34 cm² OTF in 2 mL Glandosane pH=5.9 (25 °C) | 3.34 cm² OTF in 2 mL Glandosane pH=7.40 (26,5 °C) | pH=7.2 (33 °C) |
| | 3.34 cm² OTF in 2 mL Humanspeichel pH=6.0 (25 °C) | | 3.34 cm² OTF in 2 mL Humanspeichel: pH=7,45 (22°C) pH=7.28 (33,5 °C) |

Die oralen Dünnfilme mit den Formulierungen gemäß Tabelle 6 wurden analog zu Beispiel 2 bzw. Beispiel 3 hergestellt.

### Beispiel 6

**Tabelle 7:**

| | Formulierung 12 [Gew.-%] | Formulierung 13 [Gew.-%] |
|---|---|---|
| PVA 4-88 | 39,5 | 36,15 |
| PVA 40-88 | --- | 36,15 |
| (S)-Ketamin-HCl | 50,0 | --- |
| Saccharin-Natrium | 1,0 | 1,0 |
| Sucralose | 2,0 | 2,0 |
| Cherry Flavor | 3,0 | --- |
| Glycerol | 4,5 | 4,5 |
| TRIS | --- | 20,0 |
| FD&C red 40 | --- | 0,2 |
| Flächengewicht / Größe | 193 g/m² 3,34 cm² | 160 g/m² |
| Gas | Stickstoff | Stickstoff |
| Prozesslösemittel | Wasser | Wasser |

Es wurde jeweils ein oraler Dünnfilm gemäß den Formulierungen in Tabelle 7 hergestellt. Die Herstellung erfolgte analog zu Beispiel 1 bzw. Beispiel 2.

Die oralen Dünnfilme wurden jeweils alleine in Wasser bzw. in Glandosan (künstlicher Speichel) gelöst und der jeweilig pH-Wert der Lösung bestimmt. Ferner wurden beide Filme zusammen in Wasser bzw. in Glandosan gelöst und der pH-Wert der Lösung bestimmt (siehe Tabelle 8)

**Tabelle 8:**

| | pH in Wasser (2 mL) | pH in Glandosan (2mL) |
|---|---|---|
| KetaminOTF | 5,85 | 5,11 |
| TRIS-OTF | 9,65 | 8,91 |
| KetaminOTF+TRIS-OTF | 7,13 | 6,94 |

### Beispiel 7

**Tabelle 9:**

| | Formulierung (Menge in Gew.-%) | | |
|---|---|---|---|
| Material | Formulierung 1 Wirkstoffschicht | Formulierung 7 Pufferschicht | Formulierung 14 Klebeschicht |
| PVA 4-88 | 39,1 | 39,5 | --- |
| TRIS | --- | 50,0 | --- |
| Saccharin Na | 1,0 | 1,0 | --- |
| Sucralose | 2,0 | 2,0 | --- |
| Geschmacksstoff | 3,0 | 3,0 | |
| Glycerol | 4,5 | 4,5 | 20,0 |
| S-Ketamin HCI | 50,0 | --- | --- |
| Farbstoff | 0,4 | --- | --- |
| Kollidon VA 64 | --- | --- | 80,0 |
| Gas | Luft (alternativ Stickstoff) | Luft (alternativ Stickstoff) | - (nicht geschäumt) |
| Prozesslösemittel | Wasser | Wasser | Ethanol |
| Flächengewicht (incl. Rechstfeuchtigkeit) | 190.0 g/m² | 2 x 113.0 g/m² | 2 x 63.1 g/m² |

| | | | |
|---|---|---|---|
| PVA 4-88: Polyvinylalkohol Geschmacksstoff: MANE Cherry Flavor M55394 Farbstoff: FD&C Red Kollidon VA 64: Vinylpyrrolidon-Vinylacetat-Copolymer | | | |

Es wurden jeweils Dünnfilme als Wirkstoffschicht, Pufferschicht und als Klebeschicht wie folgt hergestellt. Die Schichten wurden wie in Beispiel 1 bzw. Beispiel 2 beschrieben hergestellt. Die Zusammensetzung ist in den entsprechen Tabellen beschrieben.

Daraus wurde wie folgt ein mehrschichtiger oraler Dünnfilm mit dem folgenden Aufbau Pufferschicht-Klebeschicht-Wirkstoffschicht-Klebeschicht-Pufferschicht hergestellt. Auf ein Ketaminlaminat wurde beitseitig eine Klebeschicht auflaminiert. Nach Abziehen der Trägerfolie wurde dann beidseitig eine TRIS-Pufferschicht auflaminiert.

Es resultierte ein homogener Schaumverbund mit guten haptischen Eigenschaften und einer für die Dicke guten Auflösegeschwindigkeit.

Eine pH Messung ergab:
3,34 cm² OTF in 2 mL Wasser: pH=8,07 (bei 35,5 °C).
pH in 2 mL Humanspeichel: pH=8,04 (bei 36,3 °C).

### Beispiel 8

**Tabelle 10:**

| | Formulierung (Menge in Gew.-%) | | |
|---|---|---|---|
| Material | Formulierung 1 Wirkstoffschicht | Formulierung 7 Pufferschicht | Formulierung 14 Klebeschicht |
| PVA 4-88 | 39,1 | 39,5 | --- |
| TRIS | --- | 50,0 | --- |
| Saccharin Na | 1,0 | 1,0 | --- |
| Sucralose | 2,0 | 2,0 | --- |
| Geschmacksstoff | 3,0 | 3,0 | |
| Glycerol | 4,5 | 4,5 | 20,0 |
| S-Ketamin HCI | 50,0 | --- | --- |
| Farbstoff | 0,4 | --- | --- |
| Kollidon VA 64 | --- | --- | 80,0 |
| Gas | Luft (alternativ Stickstoff) | Luft (alternativ Stickstoff) | - (nicht geschäumt) |
| Prozesslösemittel | Wasser | Wasser | Ethanol |

| | | | |
|---|---|---|---|
| PVA 4-88: Polyvinylalkohol Geschmacksstoff: MANE Cherry Flavor M55394 Farbstoff: FD&C Red Kollidon VA 64: Vinylpyrrolidon-Vinylacetat-Copolymer | | | |

Es wurden jeweils Dünnfilme als Wirkstoffschicht, Pufferschicht und als Klebeschicht wie in den Beispielen 1,2 und 7 beschrieben hergestellt. Die Zusammensetzung ist in den entsprechenden Tabellen beschrieben.

Daraus wurde wie folgt ein mehrschichtiger oraler Dünnfilm mit dem Aufbau Pufferschicht-Klebeschicht-Wirkstoffschicht hergestellt. Auf einen Ketaminschaum wurde eine Klebeschicht auflaminiert. Nach dem Entfernen der Trägerfolie der Kleberschicht wurde ein TRIS-Schaum auflaminiert.

Es resultierte ein homogener Schaumverbund mit guten haptischen Eigenschaften und einer für die Dicke guten Auflösegeschwindigkeit.

Eine pH Messung ergab: 3,34 cm² OTF in 2 mL Wasser: pH=7,97 (bei 36,3 °C)

### Beispiel 9

**Tabelle 11**

| | Formulierung (Menge in Gew.-%) | | |
|---|---|---|---|
| Material | Formulierung 1 Wirkstoffschicht | Formulierung 5 Pufferschicht | Formulierung 14 Klebeschicht |
| PVA 4-88 | 39,1 | 64,0 | --- |
| TRIS | --- | 25,5 | --- |
| Saccharin Na | 1,0 | 1,0 | --- |
| Sucralose | 2,0 | 2,0 | --- |
| Geschmacksstoff | 3,0 | 3,0 | |
| Glycerol | 4,5 | 4,5 | 20,0 |
| S-Ketamin HCI | 50,0 | --- | --- |
| Farbstoff | 0,4 | --- | --- |
| Kollidon VA 64 | --- | --- | 80,0 |
| Gas | Luft (alternativ Stickstoff) | Luft (alternativ Stickstoff) | - (nicht geschäumt) |
| Prozesslösemittel | Wasser | Wasser | Ethanol |
| Flächengewicht (incl. Restfeuchtigkeit) | 187.2 g/m² | 216.9 g/m² | 78.4 g/m² |

| | | | |
|---|---|---|---|
| PVA 4-88: Polyvinylalkohol Geschmacksstoff: MANE Cherry Flavor M55394 Farbstoff: FD&C Red Kollidon VA 64: Vinylpyrrolidon-Vinylacetat-Copolymer | | | |

Es wurden jeweils Dünnfilme als Wirkstoffschicht, Pufferschicht und als Klebeschicht wie in den Beispielen 1,2 und 7 beschrieben hergestellt. Die Zusammensetzung ist in den entsprechenden Tabellen beschrieben.

Daraus wurde wie folgt ein mehrschichtiger oraler Dünnfilm mit dem Aufbau Pufferschicht-Klebeschicht-Wirkstoffschicht hergestellt. Auf einen Ketaminschaum wurde eine Klebeschicht auflaminiert. Nach dem Entfernen der Trägerfolie der Kleberschicht wurde ein TRIS-Schaum auflaminiert.

Es resultierte ein homogener Schaumverbund mit guten haptischen Eigenschaften und einer für die Dicke guten Auflösegeschwindigkeit.

### Beispiel 10

**Tabelle 12**

| | Formulierung (Menge in Gew.-%) | | |
|---|---|---|---|
| Material | Formulierung 1 Wirkstoffschicht | Formulierung 15 Pufferschicht | Formulierung 14 Klebeschicht |
| PVA 4-88 | 39,1 | 69,5 | --- |
| TRIS | --- | 20,0 | --- |
| Saccharin Na | 1,0 | 1,0 | --- |
| Sucralose | 2,0 | 2,0 | --- |
| Geschmacksstoff | 3,0 | 3,0 | |
| Glycerol | 4,5 | 4,5 | 20,0 |
| S-Ketamin HCI | 50,0 | --- | --- |
| Farbstoff | 0,4 | --- | --- |
| Kollidon VA 64 | --- | --- | 80,0 |
| Gas | nicht geschäumt | nicht geschäumt | nicht geschäumt |
| Prozesslösemittel | Wasser | Wasser | Ethanol |
| Flächengewicht (incl. Restfeuchtigkeit) | 187.2 g/m² | 216.9 g/m² | 78.4 g/m² |

| | | | |
|---|---|---|---|
| PVA 4-88: Polyvinylalkohol Geschmacksstoff: MANE Cherry Flavor M55394 Farbstoff: FD&C Red Kollidon VA 64: Vinylpyrrolidon-Vinylacetat-Copolymer | | | |

Es wurden jeweils Dünnfilme als Wirkstoffschicht, Pufferschicht und als Klebeschicht wie in den Beispielen 1,2 und 7 beschrieben hergestellt, wobei der Schäumungsschritt ausgelassen wurde. Die Zusammensetzung ist in den entsprechenden Tabellen beschrieben.

Daraus wurde wie folgt ein mehrschichtiger oraler Dünnfilm mit dem Aufbau Pufferschicht-Klebeschicht-Wirkstoffschicht hergestellt. Auf einen Ketaminschaum wurde eine Klebeschicht auflaminiert. Nach dem Entfernen der Trägerfolie der Kleberschicht wurde ein TRIS-Schaum auflaminiert.

Es resultierte ein homogener Schaumverbund mit guten haptischen Eigenschaften und einer für die Dicke guten Auflösegeschwindigkeit.

Eine pH Messung ergab: 3,34 cm² OTF in 2 mL Wasser: pH=6,33 (bei 32 °C).

### Beispiel 11

**Tabelle 13:**

| | Formulierung (Menge in Gew.-%) | | |
|---|---|---|---|
| Material | Formulierung 12 Wirkstoffschicht | Formulierung 3 Pufferschicht | Formulierung 16 Klebeschicht |
| PVA 4-88 | 39,5 | 69,3 | 30,0 |
| TRIS | --- | 20,0 | --- |
| Saccharin Na | 1,0 | 1,0 | --- |
| Sucralose | 2,0 | 2,0 | --- |
| Geschmacksstoff | 3,0 | 3,0 | --- |
| Glycerol | 4,5 | 4,5 | 10,0 |
| S-Ketamin HCI | 50,0 | --- | --- |
| Kollidon VA 64 | --- | --- | 28,0 |
| Sorbitol | --- | --- | 22,0 |
| Isomalt | --- | --- | 10,0 |
| Gas | Luft (alternativ Stickstoff) | Luft (alternativ Stickstoff) | Luft (alternativ Stickstoff) |
| Prozesslösemittel | Wasser | Wasser | Wasser |

| | | | |
|---|---|---|---|
| PVA 4-88: Polyvinylalkohol Geschmacksstoff: MANE Cherry Flavor M55394 Farbstoff: FD&C Red | | | |

Es wurden jeweils Dünnfilme als Wirkstoffschicht, Pufferschicht und als Klebeschicht wie in den Beispielen 1,2 und 7 beschrieben hergestellt. Die Zusammensetzung ist in den entsprechen Tabellen beschrieben.

Daraus wurde wie folgt ein mehrschichtiger oraler Dünnfilm mit dem Aufbau Pufferschicht-Klebeschicht-Wirkstoffschicht hergestellt. Auf einen Ketaminschaum wurde eine Klebeschicht auflaminiert. Nach dem Entfernen der Trägerfolie der Kleberschicht wurde ein TRIS-Schaum auflaminiert.

Es resultierte ein homogener Schaumverbund mit guten haptischen Eigenschaften und einer für die Dicke guten Auflösegeschwindigkeit.

### Beispiel 12

**Tabelle 14:**

| | Formulierung (Menge in Gew.-%) | | | | |
|---|---|---|---|---|---|
| Material | 17 | 18 | 19 | 20 | 21 |
| PVA 4-88 | 39,50 | 35,2 | 57,7 | 70,2 | 63,6 |
| Koffein | 21,83 | --- | --- | --- | --- |
| Citronensäure | 10,05 | --- | --- | --- | --- |
| Natriumcitrat-Dihydrayt | 18,12 | --- | --- | --- | --- |
| Trinatriumcitrat | --- | 54,3 | --- | --- | --- |
| Di-Kaliumhydrogenphosphat | --- | --- | 31,8 | --- | --- |
| Di-Natriumhydrogenphosphat | --- | --- | --- | --- | 25,9 |
| Natriumcarbonat | --- | --- | --- | 19,6 | --- |
| Saccharin Na | 1,00 | 1,00 | 1,00 | 1,00 | 1,00 |
| Sucralose | 2,00 | 2,00 | 2,00 | 2,00 | 2,00 |
| Geschmacksstoff | 3,00 | 3,00 | 3,00 | 3,00 | 3,00 |
| Glycerol | 4,50 | 4,50 | 4,50 | 4,50 | 4,50 |
| Gas | Luft | Masse nicht schäumbar | Masse nicht schäumbar | Masse nicht schäumbar | Masse nicht schäumbar |
| Prozesslösemittel | Wasser | Wasser | Wasser | Wasser | Wasser |

| | | | | | |
|---|---|---|---|---|---|
| PVA 4-88: Polyvinylakohol Geschmacksstoff: MANE Cherry Flavor M55394 | | | | | |

Die oralen Dünnfilme mit den Formulierungen gemäß Tabelle 14 wurden wie in den Beispielen 1,2 und 7 beschrieben hergestellt.

Die oralen Dünnfilme mit den Formulierungen gemäß Tabelle 14 zeigten folgende Nachteile:
Es bilden sich Klumpen und Inhomogenitäten in der Masse. Die Masse ist nicht homogen ausstreichbar und damit nicht geeignet für die Herstellung von Dünnfilmen. PVA ist als Matrixpolymer nicht kompatibel mit den in Tabelle 14 genannten Salzen /Puffern.

### Beispiel 13

**Tabelle 15:**

| | Formulierung (Menge in Gew.-%) | | | |
|---|---|---|---|---|
| Material | 22 | 23 | 24 | 25 |
| Kollicoat Smart Seal | 34,0 | 35,0 | 41,0 | 40,0 |
| Triethylcitrat | 5,0 | --- | 6,0 | 7 |
| TRIS | 50,0 | 50,0 | 40,0 | 40,0 |
| Saccharin Na | 2,0 | 1,0 | 1,0 | --- |
| Sucralose | 1,0 | 2,0 | 3,0 | 3,5 |
| Geschmacksstoff | 3,0 | 3,0 | 2,0 | 2,0 |
| Glycerol | --- | 7,0 | --- | --- |
| HPC EF | 5,0 | --- | 5,5 | 5,5 |
| HPMC 90SH4000 | --- | 20, | --- | --- |
| Cremophor RH40 | --- | --- | 1,5 | --- |
| Polyoxyethylen (23) laurylether | --- | --- | --- | 2,0 |
| Eigenschaften | Keine beschichtbare Masse | Laminat spröde; kein homogener Film | Masse nicht homogen; Laminat brüchig | Phasentrennung in der Masse; nicht beschichtbar |

Die oralen Dünnfilme mit den Formulierungen gemäß Tabelle 15 wurden wie in den Beispielen 1,2 und 7 beschrieben hergestellt.

Die oralen Dünnfilme mit den Formulierungen gemäß Tabelle 15 zeigten die in der Tabelle 15 aufgeführten nachteiligen Eigenschaften.

Die wässrige Polymerdispersion (Smart Seal) wurde vorgelegt und nacheinander Weichmacher, Geschmacksstoffe und Tenside zugegeben. Dann wurde das zweite Polymer zugefügt. Als Letztes wurde das Puffersalz eingerührt.

Die Masse wurde nach gängigen Verfahren geschäumt, beschichtet und getrocknet.

### Beispiel 14

Zusammen verabreichen von Formulierung 2 und Formulierung 12 (zwei getrennte OTFs je 3,34 cm² OTF):
pH in 2 mL Wasser bei 36,5 °C: pH= 7.15

Zusammen verabreichen von Formulierung 5 und Formulierung 12 (zwei getrennte OTFs je 3,34 cm² OTF):
Für Wasser:
pH in 2 mL Wasser Formulierung 12 bei 21 °C: pH= 5,85
pH in 2 mL Wasser Formulierung 5 bei 21 °C: pH= 9,65
pH in 2 mL Wasser Formulierung 12 + Formulierung 5 bei 21 °C: pH= 7,13

Für Glandosane (künstlicher Speichel)
pH in 2 mL Glandosane Formulierung 12 bei 21 °C: pH= 5,33
pH in 2 mL Glandosane Formulierung 5 bei 21 °C: pH= 8,91
pH in 2 mL Glandosane Formulierung 12 + Formulierung 5 bei 21 °C: pH= 6,94

Zusammen verabreichen von Formulierung 7 und Formulierung 12 (zwei getrennte OTFs je 3,34 cm² OTF):
Für Wasser:
pH in 2 mL Wasser Formulierung 12 bei 21 °C: pH= 5,85
pH in 2 mL Wasser Formulierung 7 bei 22 °C: pH= 9,61
pH in 2 mL Wasser Formulierung 12 + Formulierung 7 bei 22 °C: pH= 7,67

Für Glandosane (künstlicher Speichel)
pH in 2 mL Glandosane Formulierung 12 bei 21 °C: pH= 5,33
pH in 2 mL Glandosane Formulierung 7 bei 21 °C: pH= 9,01
pH in 2 mL Glandosane Formulierung 12 + Formulierung 7 bei 21 °C: pH= 7,24

### Beispiel 15

Zusammen verabreichen von Formulierung 13 und Formulierung 26 (Formulierung 13 OTF 2,27 cm² OTF und 2,27 cm² Formulierung 26):
Die Zusammensetzung Dextromethorphan OTF: Formulierung 26 ist in Tabelle 16 angegeben.

**Tabelle 16**

| **Material** | **Formulierung 26 [Gew.-%]** |
|---|---|
| Dextromethorphan | 43,78 % |
| PVA 4-88 | 38,00 % |
| Saccharin Na | 1,00 % |
| Sucralose | 2,00 % |
| Glycerol | 4,54 % |
| Flavor | 6,00 % |
| Amberlite IRP64 | 4,48 % |
| FD&C Red 40 | 0,20 % |
| Trometamol (TRIS) | -- |
| **Lösungsmittel** | Aqua purificata |
| **Flächengewicht** | 188 g/cm² |

Ein oraler Dünnfilm mit der Formulierung gemäß Tabelle 16 wurden wie in den Beispielen 1,2 und 7 beschrieben hergestellt.

Eine pH Messung ergab: pH in 2 mL Wasser bei 35 °C: pH= 7,63.

### Beispiel 16

Zusammen verabreichen von Formulierung 13 und Formulierung 27 (Formulierung 13 OTF 2,27 cm² OTF und 2,27 cm² Formulierung 27):
Die Zusammensetzung Paracetamol (Acetaminophen) OTF Formulierung 27 ist in Tabelle 17 angegeben.

**Tabelle 17**

| **Material** | **Formulierung 27 [Gew.-%]** |
|---|---|
| Paracetamol | 20,00 % |
| PVA 4-88 | 72,50 % |
| Saccharin Na | 0,50 % |
| Sucralose | 1,00 % |
| Glycerol | 4,50 % |
| Flavor | 1,50 % |
| Trometamol (TRIS) | -- |
| Lösungsmittel | Aqua purificata |
| Flächengewicht | 103 g/cm² |

Ein oraler Dünnfilm mit der Formulierung gemäß Tabelle 17 wurden wie in den Beispielen 1,2 und 7 beschrieben hergestellt.

Eine pH Messung ergab: pH in 2 mL Wasser bei 33 °C: pH= 8,17.

### Beispiel 18:

Zusammen verabreichen von Formulierung 13 OTF 2,72 cm² OTF und kommerziellen BEMA Fentanyl OTF (Stärke 200 µg):
Eine pH Messung ergab: pH in 2 mL Wasser bei 22 °C: pH= 8,67.

### Beispiel 19

Bestimmung des Einflusses des pH-Wertes auf die Permeation von protonierbaren/deprotonierbren Wirkstoffen. Einstellung des pH-Wertes mittels eines Puffer-OTFs kann die Varianz im pH-Wert zwischen Individuen reduzieren und den pH-Wert in einen für die Permeation bevorzugten Bereich bringen. Mehrschichtige OTFs können durch eine höhere Dissintegrations-/Auflösungzeit das Freisetzungsprofil beeinflussen.

Ketamin-OTFs mit Puffersubstanzen erzielen deutlich bessere Permeationen (mehr als 18x höher) als Ketamin·HCI (ohne OTF).

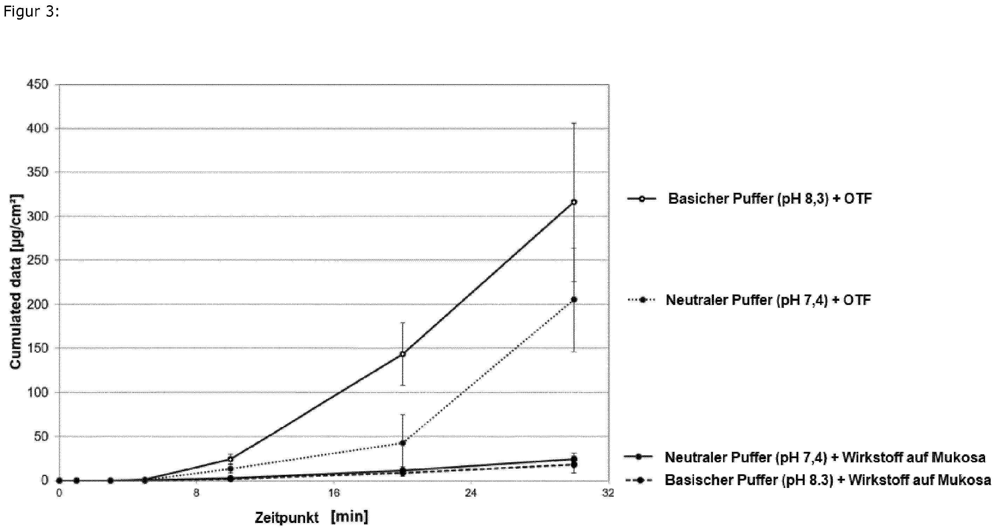

Die Ergebnisse sind in Figur 3 zusammengefasst.

## Patentansprüche

1. Oraler Dünnfilm, umfassend mindestens eine Schicht, die mindestens einen Polyvinylalkohol und Tris(hydroxymethyl)aminomethan in einer Menge von 15 bis 70 Gew.-%, bezogen auf das Gesamtgewicht der mindestens einen Schicht, enthält.

2. Oraler Dünnfilm gemäß Anspruch 1, **dadurch gekennzeichnet, dass** Polyvinylalkohol in einer Menge von 20 bis 90 Gew.-%, bezogen auf das Gesamtgewicht der mindestens einen Schicht, in der mindestens einen Schicht enthalten ist.

3. Oraler Dünnfilm gemäß irgendeinem der vorhergehenden Ansprüche, **dadurch gekennzeichnet, dass** Tris(hydroxymethyl)aminomethan in einer Menge von 25 bis 55 Gew.-%, bezogen auf das Gesamtgewicht der mindestens einen Schicht, in der mindestens einen Schicht enthalten ist.

4. Oraler Dünnfilm gemäß irgendeinem der vorhergehenden Ansprüche, **dadurch gekennzeichnet, dass** die mindestens eine Schicht mindestens einen pharmazeutisch aktiven Wirkstoff umfasst.

5. Oraler Dünnfilm gemäß irgendeinem der vorhergehenden Ansprüche, **dadurch gekennzeichnet, dass** die mindestens eine Schicht mindestens einen pharmazeutisch aktiven Wirkstoff umfasst, der ausgewählt ist aus der Gruppe, umfassend die Wirkstoffklassen der Analgetika, Hormone, Hypnotika, Sedativa, Antiepileptika, Weckamine, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narcotika, wobei der mindestens eine pharmazeutisch aktive Wirkstoff vorzugsweise Ketamin, besonders bevorzugt (S)-Ketamin, oder pharmazeutisch akzeptabler Salze daraus, umfasst.

6. Oraler Dünnfilm gemäß irgendeinem der vorhergehenden Ansprüche, **dadurch gekennzeichnet, dass** die mindestens eine Schicht mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, Feuchthaltemittel, eine Säure oder eine Base (bzw. ein Salz davon) Konservierungsmittel und/oder Antioxidationsmittel, umfasst.

7. Oraler Dünnfilm gemäß irgendeinem der vorhergehenden Ansprüche, **dadurch gekennzeichnet, dass** der orale Dünnfilm mindestens eine weitere Schicht aufweist, die mindestens ein Matrixpolymer und mindestens einen pharmazeutisch aktiven Wirkstoff umfasst.

8. Oraler Dünnfilm gemäß Anspruch 7, **dadurch gekennzeichnet, dass** das mindestens eine Polymer ein wasserlösliches Polymer ist, das ausgewählt ist aus der Gruppe umfassend Stärke und Stärkederivaten, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylate, Polyvinylpyrrolidone, Vinvlpyrrolidon/Vinylacetat-Copolymere, Polyvinylalkohole, Polyethylenoxidpolymere, Polyacrylamide, Polyethylenglycole, Gelatine, Collagen, Alginaten, Pektin, Pullulan, Traganth, Chitosan, Alginsäure, Arabinogalaktan, Galaktomannan, Agar, Agarose, Carrageen und natürlichen Gummen.

9. Oraler Dünnfilm gemäß Anspruch 7 oder 8, **dadurch gekennzeichnet, dass** der mindestens eine pharmazeutisch aktive Wirkstoff ausgewählt ist aus der Gruppe, umfassend die Wirkstoffklassen der Analgetika, Hormone, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormonen, Sexualhormonen, Antidiabetika, Antitumor-Wirkstoffen, Antibiotika, Chemotherapeutika und Narcotika, wobei der mindestens eine pharmazeutisch aktive Wirkstoff vorzugsweise Ketamin, besonders bevorzugt (S)-Ketamin, oder pharmazeutisch akzeptables Salze daraus, umfasst.

10. Oraler Dünnfilm gemäß irgendeinem der vorhergehenden Ansprüche, **dadurch gekennzeichnet, dass** die mindestens eine Schicht, die mindestens einen Polyvinylalkohol und Tris(hydroxymethyl)aminomethan enthält, und/oder die mindestens eine weitere Schicht, die mindestens ein Matrixpolymer und mindestens einen pharmazeutisch aktiven Wirkstoff umfasst, in Form eines Hohlräume aufweisenden verfestigten Schaums vorliegt (vorliegen).

11. Oraler Dünnfilm gemäß Anspruch 10, **dadurch gekennzeichnet, dass** die Hohlräume voneinander isoliert sind und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

12. Oraler Dünnfilm gemäß Anspruch 10 oder 11, **dadurch gekennzeichnet, dass** die Hohlräume miteinander in Verbindung stehen und vorzugsweise ein die Matrixschicht durchdringendes Kanalsystem bilden.

13. Oraler Dünnfilm gemäß irgendeinem der Ansprüche 10 bis 12, **dadurch gekennzeichnet, dass** die genannten Hohlräume einen Volumenanteil von 5 bis 98 %, bevorzugt von 50 bis 80 %, bezogen auf das Gesamtvolumen der jeweiligen Schicht, ausmachen.

14. Oraler Dünnfilm gemäß irgendeinem der Ansprüche 7 bis 13, **dadurch gekennzeichnet, dass** die mindestens eine Schicht, die mindestens einen Polyvinylalkohol und Tris(hydroxymethyl)aminomethan enthält, und/oder die mindestens eine weitere Schicht, die mindestens ein Matrixpolymer und mindestens einen pharmazeutisch aktiven Wirkstoff umfasst, direkt aufeinander laminiert sind oder durch eine dazwischenliegende Klebeschicht oder Trennschicht miteinander verbunden sind.

15. Oraler Dünnfilm gemäß irgendeinem der vorhergehenden Ansprüche, **dadurch gekennzeichnet, dass** der orale Dünnfilm im Mund eines Patienten einen pH-Wert (bei 37°C) von 6 bis 9, vorzugsweise von 6 bis 8, erzeugt.

16. Oraler Dünnfilm gemäß irgendeinem der vorhergehenden Ansprüche zur Verwendung als Arzneimittel.

## Claims

1. An oral thin film, comprising at least one layer, which contains at least one polyvinyl alcohol and tris(hydroxymethyl)aminomethane in an amount of from 15 to 70 wt.% in relation to the total weight of the at least one layer.

2. The oral thin film according to claim 1, **characterised in that** polyvinyl alcohol is contained in the at least one layer in an amount of from 20 to 90 wt.% in relation to the total weight of the at least one layer.

3. The oral thin film according to any one of the preceding claims, **characterised in that** tris(hydroxymethyl)aminomethane is contained in the at least one layer in an amount of from 25 to 55 wt.% in relation to the total weight of the at least one layer.

4. The oral thin film according to any one of the preceding claims, **characterised in that** the at least one layer comprises at least one pharmaceutically active agent.

5. The oral thin film according to any one of the preceding claims, **characterised in that** the at least one layer comprises at least one pharmaceutically active agent which is selected from the group comprising the active agent classes of analgesics, hormones, hypnotics, sedatives, antiepiletics, analeptics, psychoneurotropic drugs, neuro-muscle blockers, antspasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active agents, antibiotics, chemotherapeutics and narcotics, the at least one pharmaceutically active agent preferably comprising ketamine, especially preferably (S)-ketamine, or pharmaceutically acceptable salts thereof.

6. The oral thin film according to any one of the preceding claims, **characterised in that** the at least one layer comprises at least one auxiliary substance selected from the group comprising colouring agents, flavourings, sweeteners, plasticisers, taste-masking agents, emulsifiers, enhancers, humectants, an acid or a base (or a salt thereof), preservatives and/or antioxidants.

7. The oral thin film according to any one of the preceding claims, **characterised in that** the oral thin film has at least one further layer which comprises at least one matrix polymer and at least one pharmaceutically active agent.

8. The oral thin film according to claim 7, **characterised in that** the at least one polymer is a water-soluble polymer which is selected from the group comprising starch and starch derivatives, dextrans, cellulose derivatives, such as carboxymethyl cellulose, hydroxypropyl cellulose, hydroxyethyl cellulose, hydroxypropyl methylcellulose, hydroxypropyl ethyl cellulose, sodium carboxymethyl cellulose, ethyl or propyl cellulose, polyacrylic acids, polyacrylates, polyvinylpyrrolidones, vinyl pyrrolidone/vinyl acetate copolymers, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, and natural gums.

9. The oral thin film according to claim 7 or 8, **characterised in that** the at least one pharmaceutically active agent is selected from the group comprising the active agent classes of analgesics, hormones, hypnotics, sedatives, antiepiletics, analeptics, psychoneurotropic drugs, neuro-muscle blockers, antspasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active agents, antibiotics, chemotherapeutics and narcotics, the at least one pharmaceutically active agent preferably comprising ketamine, especially preferably (S)-ketamine, or pharmaceutically acceptable salts thereof.

10. The oral thin film according to any one of the preceding claims, **characterised in that** the at least one layer which contains at least one polyvinyl alcohol and tris(hydroxymethyl)aminomethane, and/or the at least one further layer which comprises at least one matrix polymer and at least one pharmaceutically active agent is present (are present) in the form of a solidified foam having voids.

11. The oral thin film according to claim 10, **characterised in that** the voids are isolated from one another and are preferably present in the form of bubbles, the voids being filled with air or a gas, preferably with an inert gas, especially preferably with nitrogen, carbon dioxide, helium or a mixture of at least two of these gases.

12. The oral thin film according to claim 10 or 11, **characterised in that** the voids are connected to one another and preferably form a channel system penetrating the matrix layer.

13. The oral thin film according to any one of claims 10 to 12, **characterised in that** said voids account for a volume fraction of from 5 to 98%, preferably from 50 to 80%, in relation to the total volume of the layer in question.

14. The oral thin film according to any one of claims 7 to 13, **characterised in that** the at least one layer which contains at least one polyvinyl alcohol and tris(hydroxymethyl)aminomethane, and/or the at least one further layer which comprises at least one matrix polymer and at least one pharmaceutically active agent are laminated directly on one another or are connected to one another by an intermediate adhesive layer or separation layer.

15. The oral thin film according to any one of the preceding claims, **characterised in that** the oral thin film in the mouth of a patient produces a pH value (at 37°C) of from 6 to 9, preferably of from 6 to 8.

16. The oral thin film according to any one of the preceding claims for use as a medicament.

## Revendications

1. Film mince oral, comprenant au moins une couche qui contient au moins un alcool polyvinylique et du tris (hydroxyméthyl)aminométhane en une quantité de 15 à 70 % en poids, par rapport au poids total de l'au moins une couche.

2. Film mince oral selon la revendication 1, **caractérisé en ce que** l'alcool polyvinylique est contenu en une quantité de 20 à 90 % en poids, par rapport au poids total de l'au moins une couche, dans l'au moins une couche.

3. Film mince oral selon l'une quelconque des revendications précédentes, **caractérisé en ce que** le tris(hydroxyméthyl)aminométhane est contenu en une quantité de 25 à 55 % en poids, par rapport au poids total de l'au moins une couche, dans l'au moins une couche.

4. Film mince oral selon l'une quelconque des revendications précédentes, **caractérisé en ce que** l'au moins une couche comprend au moins un principe actif pharmaceutique.

5. Film mince oral selon l'une quelconque des revendications précédentes, **caractérisé en ce que** l'au moins une couche comprend au moins un principe actif pharmaceutique qui est choisi dans le groupe comprenant les classes de principes actifs des analgésiques, des hormones, des hypnotiques, des sédatifs, des antiépileptiques, des weckamines, des psychoneurotropes, des bloqueurs neuromusculaires, des antispasmodiques, des antihistaminiques, des antiallergiques, des cardiotoniques, des antiarythmiques, des diurétiques, des hypotenseurs, des vasopresseurs, des antidépresseurs, des antitussifs, des expectorants, des hormones thyroïdiennes, des hormones sexuelles, des antidiabétiques, des principes actifs antitumoraux, des antibiotiques, des chimiothérapeutiques et des narcotiques, dans lequel l'au moins un principe actif pharmaceutique comprend de préférence la kétamine, de manière particulièrement préférée la (S)-kétamine, ou des sels pharmaceutiquement acceptables de celle-ci.

6. Film mince oral selon l'une quelconque des revendications précédentes, **caractérisé en ce que** l'au moins une couche comprend au moins un excipient choisi dans le groupe comprenant les colorants, les arômes, les édulcorants, les plastifiants, les agents de masquage du goût, les émulsifiants, les exhausteurs, les humectants, un acide ou une base (ou un sel de ceux-ci), les conservateurs et/ou les antioxydants.

7. Film mince oral selon l'une quelconque des revendications précédentes, **caractérisé en ce que** le film mince oral présente au moins une autre couche qui comprend au moins un polymère matriciel et au moins un principe actif pharmaceutique.

8. Film mince oral selon la revendication 7, **caractérisé en ce que** l'au moins un polymère est un polymère soluble dans l'eau, qui est choisi dans le groupe comprenant l'amidon et les dérivés d'amidon, les dextranes, les dérivés de cellulose, tels que la carboxyméthylcellulose, l'hydroxypropylcellulose, l'hydroxyéthylcellulose, l'hydroxypropylméthylcellulose, l'hydroxypropyléthylcellulose, la carboxyméthylcellulose de sodium, l'éthylcellulose ou la propylcellulose, les acides polyacryliques, les polyacrylates, la polyvinylpyrrolidone, les copolymères de vinylpyrrolidone/acétate de vinyle, les alcools polyvinyliques, les polymères d'oxyde de polyéthylène, les polyacrylamides, les polyéthylènes glycol, la gélatine, le collagène, les alginates, la pectine, le pullulane, la gomme adragante, le chitosan, l'acide alginique, l'arabinogalactane, le galactomannane, la gélose, l'agarose, le carraghénane et les gommes naturelles.

9. Film mince oral selon la revendication 7 ou 8, **caractérisé en ce que** l'au moins un principe actif pharmaceutique est choisi dans le groupe comprenant les classes de principes actifs des analgésiques, des hormones, des hypnotiques, des sédatifs, des antiépileptiques, des weckamines, des psychoneurotropes, des bloqueurs neuromusculaires, des antispasmodiques, des antihistaminiques, des antiallergiques, des cardiotoniques, des antiarythmiques, des diurétiques, des hypotenseurs, des vasopresseurs, des antidépresseurs, des antitussifs, des expectorants, des hormones thyroïdiennes, des hormones sexuelles, des antidiabétiques, des principes actifs antitumoraux, des antibiotiques, des chimiothérapeutiques et des narcotiques, dans lequel l'au moins un principe actif pharmaceutique comprend de préférence la kétamine, de manière particulièrement préférée la (S)-kétamine, ou des sels pharmaceutiquement acceptables de celle-ci.

10. Film mince oral selon l'une quelconque des revendications précédentes, **caractérisé en ce que** l'au moins une couche, qui contient au moins un alcool polyvinylique et du tris (hydroxyméthyl)aminométhane, et/ou l'au moins une autre couche, qui comprend au moins un polymère matriciel et au moins un principe actif pharmaceutique, est présente (sont présentes) sous forme d'une mousse solidifiée présentant des cavités.

11. Film mince oral selon la revendication 10, **caractérisé en ce que** les cavités sont isolées les unes des autres et se présentent de préférence sous la forme de bulles, dans lequel les cavités sont remplies d'air ou d'un gaz, de préférence d'un gaz inerte, de manière particulièrement préférée d'azote, de dioxyde de carbone, d'hélium ou d'un mélange d'au moins deux de ces gaz.

12. Film mince oral selon la revendication 10 ou 11, **caractérisé en ce que** les cavités sont reliées entre elles et forment de préférence un système de canaux pénétrant la couche de matrice.

13. Film mince oral selon l'une quelconque des revendications 10 à 12, **caractérisé en ce que** lesdites cavités représentent un pourcentage volumique de 5 à 98 %, de préférence de 50 à 80 %, par rapport au volume total de la couche respective.

14. Film mince oral selon l'une quelconque des revendications 7 à 13, **caractérisé en ce que** l'au moins une couche, qui contient au moins un alcool polyvinylique et du tris(hydroxyméthyl)aminométhane, et/ou l'au moins une autre couche, qui comprend au moins un polymère matriciel et au moins un principe actif pharmaceutique, sont stratifiées directement l'une sur l'autre ou sont reliées entre elles par une couche adhésive ou couche de séparation intermédiaire.

15. Film mince oral selon l'une quelconque des revendications précédentes, **caractérisé en ce que** le film mince oral produit dans la bouche d'un patient un pH (à 37 °C) de 6 à 9, de préférence de 6 à 8.

16. Film mince oral selon l'une quelconque des revendications précédentes destiné à être utilisé comme médicament.
